# EUROPÄISCHE PATENTSCHRIFT

(11) **EP 2 554 535 B1**
(45) Veröffentlichungstag und Bekanntmachung des Hinweises auf die Patenterteilung: **16.01.2019**
(21) Anmeldenummer: 11176426.2
(22) Anmeldetag: 03.08.2011
(51) Int. Cl.: C07C 67/08, C07C 69/54

(54) **Verfahren zur Herstellung von (Meth)acrylsäureestern von Polyolen**
Method for manufacturing (meth)acrylic acid esters from polyols
Procédé de fabrication d'esters d'acide (méth)acrylique de polyoles

(43) Veröffentlichungstag der Anmeldung: 06.02.2013
(73) Patentinhaber: Cognis IP Management GmbH, 40589 Düsseldorf (DE)
(72) Erfinder: Baro, Juergen, 73732 Esslingen (DE); Ballin, Jean-Marc, 93160 Noisy le Grand (FR)
(74) Vertreter: BASF IP Association

(56) Entgegenhaltungen:
- EP-A1- 0 890 568
- GB-A- 1 489 536
- US-A- 4 187 383

## Beschreibung

### Gebiet der Erfindung

Die Erfindung betrifft ein neues Verfahren zur Herstellung von (Meth)acrylsäureestern mehrwertiger Alkohole (Polyole).

### Stand der Technik

(Meth)acrylsäureester mehrwertiger Alkohole insbesondere aus der Gruppe der 2-bis 6-wertigen aliphatischen gesättigten Alkohole und deren Oxalkylierungsprodukten finden zunehmende Bedeutung als hochreaktive Bestandteile in strahlenhärtenden Systemen. Solche polyfunktionellen (Meth)acrylsäureester können beispielsweise als Lackrohstoffe für die Elektronenstrahlhärtung oder als Bestandteil von UV-härtenden Druckfarben oder entsprechenden Überzugslacken, Spachtel, Form- oder Vergussmassen sowie in Klebstoffen, insbesondere anaerob härtenden Klebstoffen Verwendung finden. Ihre Herstellung ist allerdings nicht problemlos. Gefordert werden insbesondere farblose Produkte mit geringer Säurezahl und hoher Lagerstabilität, die praktisch auch keinen Eigengeruch aufweisen. Eine destillative Reinigung der (Meth)acrylsäureester der hier betroffenen Art scheidet in aller Regel aufgrund ihres hohen Molekulargewichtes und ihrer hohen Reaktivität aus. Die Produkte sollen also unmittelbar als möglichst farblose Reaktionsprodukte der Veresterung anfallen. Die Durchführung der Veresterungsreaktion fordert die Mitverwendung hochwirksamer Inhibitoren, die ihrerseits keine unerwünschten Nebenreaktionen, beispielsweise Verfärbungen, auslösen.

Das dominierende industrielle Produktionsverfahren für die direkte Veresterung von (Meth)Acrylsäure mit Hydroxyverbindungen basiert auf der Verwendung von flüchtigen organischen Lösemitteln als flüssigem Reaktionsmedium, auch als Lösemittelprozess bekannt. Geeignete flüchtige organische Lösemittel sind beispielsweise Toluol, Cyclohexan, Methylcyclohexan oder n-Heptan, die zudem als azeotropes Schleppmittel zur kontinuierlichen Ausschleusung des entstehenden Reaktionswassers aus dem Reaktionsgemisch genutzt und nach Reaktionsende destillativ wieder abgetrennt werden. Entsprechende Verfahrensbeschreibungen finden sich beispielsweise in US-6,838,515 und EP-A-127,766. Trotz der destillativen Entfernung der flüchtigen organischen Lösemittel nach Reaktionsende verbleiben in so hergestellten Monomer- und Oligomer-(Meth)Acrylaten jedoch stets Restlösemittelspuren, die deren Mengen sich im Bereich von 50 - 10000 ppm bewegen.

Da die Verwendung von organischen Lösemitteln aus Umweltschutzgesichtspunkten zunehmend reglementiert wird, gewinnt ein alternatives Produktionsverfahren an Bedeutung, bei dem die direkte Veresterung von (Meth)Acrylsäure mit Hydroxyverbindungen in (Meth)Acrylsäure selbst als flüssigem Reaktionsmedium durchgeführt wird ohne Verwendung flüchtiger organischer Lösemittel, ein Verfahren, das auch als lösemittelfreier Prozess bekannt ist. Dabei wird das entstehende Reaktionswasser aus dem Reaktionsgemisch als Wasser/(Meth)Acrylsäure-Gemisch destillativ ausgekreist. Entsprechende Verfahren sind beispielsweise in EP-B-449 919 und EP-B-1 204 472 beschrieben. Auf diesem Wege lassen sich vollständig lösemittelfreie Monomer- und Oligomer-(Meth)Acrylate herstellen, die unter anderem dort eingesetzt werden, wo Spuren von flüchtigen organischen Lösemitteln einen unerwünschten Eigengeruch wie beispielsweise im Verpackungsbereich entfalten können.

Beide Produktionstechnologien wurden im Laufe der Jahre kontinuierlich weiterentwickelt, wobei die Schwerpunkte auf der Optimierung des Durchsatzes und der Minimierung des Rohstoffeinsatzes lagen. Die resultierende Reinheit der auf diesem Wege hergestellten Monomer- und Oligomer-(Meth)Acrylate hat dagegen bislang wenig Beachtung gefunden. Unter der Reinheit wird in der einschlägigen Fachliteratur in der Regel der Gesamtgehalt an vollständig und partiell (meth)acrylierten Substanzspezies im Endprodukt verstanden, der aus dem Restgehalt an nicht umgesetzten Hydroxyverbindungen berechnet wird und so meist über 97% (GC-Flächenprozent) liegt.

Diese weitverbreitete Definition des Begriffes Reinheit sagt jedoch streng genommen nichts über den tatsächlich vorhandenen Gehalt an der gewünschten, vollständig (meth)acrylierten Substanzspezies im Endprodukt aus und ist daher unbefriedigend. Im Rahmen der vorliegenden Erfindung wird daher unter "Reinheit" der Gehalt von (Meth)acrylsäureestern an vollständig (meth)acrylierter Substanzspezies verstanden.Dabei ist unter vollständig (meth)acrylierter Substanzspezies zu verstehen, dass darin sämtliche OH-Gruppen des Alkoholbausteins des (Meth)acrylsäureesters in veresterter Form vorliegen.
Diese im Rahmen der vorliegenden Erfindung strikt geltende Definition des Begriffes Reinheit ist insbesondere vor dem Hintergrund von besonderer Bedeutung, dass bei polyfunktionellen Monomer- und Oligomer-(Meth)Acrylaten unter REACH (Registration, Evaluation, Authorisation and Restriction of Chemicals) die Substanzidentifikation und -klassifizierung ausschließlich über CAS-Nummern mit entsprechenden Reinheitsanforderungen bezogen auf die jeweilige vollständig (meth)acrylierte Substanzspezies erfolgt (siehe ECHA-Publikation "Guidance for Identification and Naming of Substances under REACH - June 2007"). So werden für sogenannte "mono-constituent substances" unter REACH, zu denen beispielsweise 1.6-Hexandioldiacrylat, Tripropylenglykoldiacrylat und Trimethylolpropantriacrylat gehören, Reinheiten von mindestens 80% - bezogen auf die gewünschte, vollständig acrylierte Substanzspezies - im Endprodukt gefordert.
Aus der Fachliteratur ist bekannt, dass beispielsweise polyfunktionelle Monomeracrylate wie 1.6-Hexandioldiacrylat, Tripropylenglykoldiacrylat, Trimethylolpropantriacrylat und Pentaerythrittetraacrylat Mischungen aus Substanzspezies mit unterschiedlichen Acrylierungsgraden und anderen Nebenprodukten darstellen, bei denen die gewünschte, vollständig acrylierte Substanzspezies nur ein Teil der Mischung ist (vergleiche R. H. Hall, F. P. B. Van Der Maeden, A. C. C. M. Willemsen, Spec. Chem., 7, 56-64 (1987) und M. Matsunaga, Y. Matsushima, H. Ohtani, S. Tsuge, Anal. Sci., 17, 1295-1299 (2001)).

Durch noch komplexere Zusammensetzungen sind solche polyfunktionelle Monomer- und Oligomer-(Meth)acrylate gekennzeichnet, die auf Polyhydroxyverbindungen basieren, die mit Ethylenoxid und/oder Propylenoxid alkoxyliert sind. Solche Produkte weisen verfahrensbedingt stets eine Verteilungskurve mit unterschiedlichen Alkoxylierungsgraden um einen Mittelwert auf, so dass die Zahl der möglichen vollständig und partiell (meth)acrylierten Substanzspezies nicht nur von der Zahl der verfügbaren Hydroxygruppen abhängig ist, sondern auch noch multiplikativ mit der Zahl der unterschiedlich alkoxylierten Polyhydroxyspezies verbunden ist.

Entsprechende Untersuchungen zur Zusammensetzung solcher polyfunktionellen, alkoxylierten Monomer- und Oligomeracrylate wie ethoxyliertes 1.6-Hexandioldiacrylat, propoxyliertes Neopentylglykoldiacrylat, ethoxyliertes Trimethylolpropantriacrylat, propoxyliertes Glycerintriacrylat und Polypropylenglykoldiacrylat finden sich bei T. Marek, U. Gröllman, DIC Technical Review, No. 5, 85-93 (1999), M. Matsunaga, Y. Matsushima, H. Yokoi, H. Ohtani, S. Tsuge, Anal. Sci., 18, 277-281 (2002) und S. J. Yoo, G. V. Pace, B. K. Khoo, J. Lech, T. G. Hartman, RadTech Report, May/June, 60-68 (2004).

Die sauer katalysierte, direkte Veresterung von (Meth)Acrylsäure mit Monohydroxyverbindungen ist in erster Näherung noch als einfache Phasengleichgewichtreaktion zu betrachten, bei der das Phasengleichgewicht durch die kontinuierliche Entfernung des Reaktionswassers aus dem Reaktionsgemisch über die geeignete Wahl von Temperatur, Druck und Reaktionszeit nahezu vollständig auf die Produktseite verschoben werden kann.

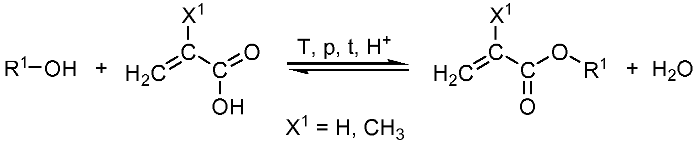

Das Endprodukt enthält in der Regel die gewünschte, vollständig (meth)acrylierte Sustanzspezies in Reinheiten größer 97%.

Für Polyhydroxyverbindungen ist der Verlauf der sauer katalysierten, direkten Veresterung von (Meth)Acrylsäure dagegen wesentlich komplexer wie bei L.-D. Shiau, T.-R. Ling, D.-S. Tseng, Chem. Eng. Comm., 179, 133-148 (2000) beschrieben, da verschiedene, miteinander gekoppelte Phasengleichgewichtsreaktionen nebeneinander ablaufen, die je nach Reaktionsführung auch zu substantiellen Mengen an partiell (meth)acrylierten Monomer- oder Oligomer-(Meth)Acrylaten im Endprodukt führen können.

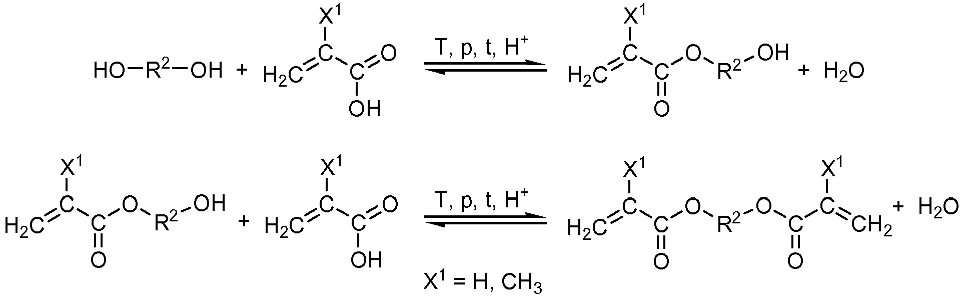

Außerdem können, wie von R. H. Hall, F. P. B. Van Der Maeden, A. C. C. M. Willemsen, Spec. Chem., 7, 56-64 (1987) dargelegt, diese partiell (meth)acrylierten Monomer- oder Oligomer-(Meth)Acrylate - wie auch nicht umgesetzte (Meth)Acrylsäure und nicht umgesetzte Polyhydroxyverbindungen - Nebenreaktionen wie Michael-Additionen mit der vollständig (meth)acrylierten Substanzspezies sowie untereinander eingehen.

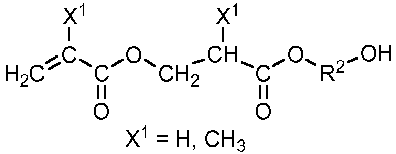

### Michael-Addukt Mono(meth)acrylat/(Meth)Acrylsäure

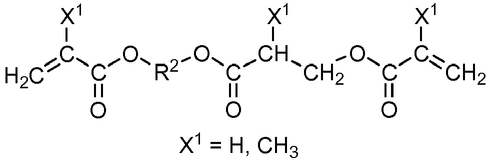

### Michael-Addukt Di(meth)acrylat/(Meth)Acrylsäure

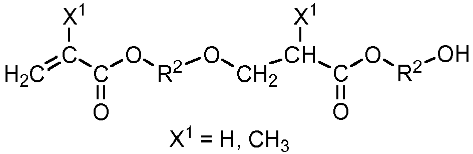

### Michael-Addukt Mono(meth)acrylat/Mono(meth)acrylat

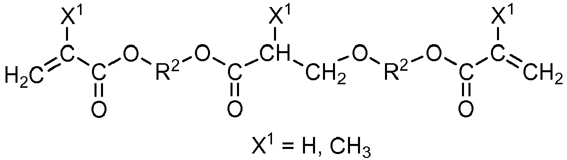

### Michael-Addukt Di(meth)acrylat/Mono(meth)acrylat

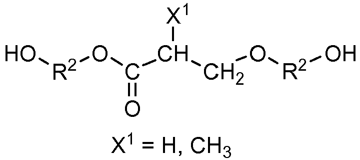

### Michael-Addukt Mono(meth)acrylat/Diol

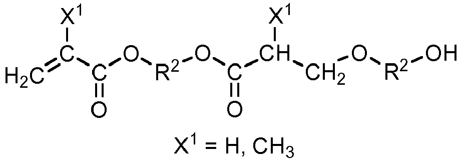

### Michael-Addukt Di(meth)acrylat/Diol

Das Vorliegen von partiell (meth)acrylierten Monomer- oder Oligomer-(Meth)Acrylaten sowie von Michael-Addukten im Endprodukt führt zwangsläufig zu einem erheblichen Verlust an Reinheit bezogen auf die gewünschte, vollständig (meth)acrylierte Substanzspezies.

Obgleich viele bekannte Monomeracrylate wie 1.6-Hexandioldiacrylat, Tripropylenglykoldiacrylat, Trimethylolpropantriacrylat oder Pentaerythrittetraacrylat mehr als 97% an acrylierten Substanzspezies enthalten, so liegt ihr Gehalt an der gewünschten, vollständig acrylierten Substanzspezies gleichwohl häufig signifikant unterhalb von 80%. Die unerwünschten Nebenprodukte wie Michael-Addukte oder partiell acrylierte Substanzspezies verursachen dabei nicht nur eine verminderte Reinheit, sondern können auch das Eigenschaftsprofil erheblich beeinflussen. So führen partiell (meth)acrylierte Monomer- und Oligomer-(Meth)Acrylate zu höheren Viskositäten aufgrund der Bildung von Wasserstoffbrückenbindungen über ihre freien Hydroxygruppen sowie zu Einbußen bei der Reaktivität bedingt durch fehlende Doppelbindungen. Michael-Addukte bewirken aufgrund ihres hohen Molekulargewichtes ebenfalls einen Viskositätsanstieg und senken die Doppelbindungsdichte, da zu ihrer Bildung Doppelbindungen verbraucht werden.

### Beschreibung der Erfindung

Aufgabe der vorliegenden Erfindung war es vor dem Hintergrund der oben geschilderten Zusammenhänge, ein neues Verfahren zur Herstellung von lösemittelfreien (Meth)acrylsäureestern mehrwertiger Alkohole bereitzustellen; dabei sollten die durch das neue Verfahren zugänglichen (Meth)acrylsäureester die Maßgabe erfüllen, dass mindestens 80 mol-% der in den eingesetzten Polyolen vorhandenen OH-Gruppen in veresterter Form vorliegen, was einer Reinheit von mindestens 80% im Sinne der oben dargelegten Definition entspricht.

Überraschenderweise wurde gefunden, dass ein portionsweises Zugeben von (Meth)acrylsäure zu Polyolen mit nachfolgendem oder gleichzeitigem Ausschleusen des bei der Veresterung gebildeten Reaktionswassers zu einer Reinheit von mindestens 80% - bezogen auf die gewünschte, vollständig acrylierte Substanzspezies im Endprodukt - führt, wobei spezielle Parameter einzuhalten sind.

Gegenstand der vorliegenden Erfindung ist ein Verfahren zur Herstellung von (Meth)acrylsäureestern von Polyolen, wobei der Gehalt dieser Ester an Species, bei denen sämtliche OH-Gruppen der Polyole verestert sind, 80 Mol% oder mehr beträgt, durch Umsetzung von Polyolen mit Acrylsäure und/oder Methacrylsäure in Gegenwart von sauren Veresterungskatalysatoren und in Gegenwart von Polymerisationsinhibitoren, wobei man mit bei Reaktionstemperatur flüssigen Reaktionsgemischen arbeitet, die frei von nicht reagierenden Lösungs- und/oder azeotropen Schleppmitteln sind, wobei das entstehende Kondensationswasser aus der Gasphase des Reaktionsraumes abgezogen wird, dadurch gekennzeichnet, dass man (Meth)Acrylsäure in 4 bis 16 Portionen zudosiert, wobei folgende Massgaben gelten:
- die Menge der einzelnen (Meth)acrylsäure-Portionen wird jeweils im Bereich von 5 bis 40 Mol-% - bezogen auf die Gesamtheit der OH-Gruppen der eingesetzten Polyole - eingestellt,
- die Anzahl der (Meth)acrylsäure-Portionen multipliziert mit der Menge der eingesetzten (Meth)acrylsäure-Portionen (in Mol%) ergibt einen Wert von mindestens 100 (mol%),
- die Reaktionstemperatur wird auf einen Wert im Bereich von 70 bis 150 °C eingestellt und
- das bei der Reaktion entstehende Wasser wird unter reduziertem Druck aus dem Reaktionsraum entfernt, wobei der reduzierte Druck bei 600 hPa oder weniger liegt.

Unter "nicht reagierenden Lösungs- und/oder azeotropen Schleppmitteln" sind solche Lösungsmittel bzw. azeotropen Schleppmittel zu verstehen, die unter den Reaktionsbedingungen des erfindungsgemäßen Verfahrens chemisch inert sind. Die obige Angabe "wobei man mit bei Reaktionstemperatur flüssigen Reaktionsgemischen arbeitet, die frei von nicht reagierenden Lösungs- und/oder azeotropen Schleppmitteln sind" bedeutet mithin, dass beim erfindungsgemäßen Verfahren auf derartige Lösungsmittel oder azeotrope Schleppmittel verzichtet wird.

Wie ausgeführt erfolgt die Zudosierung von (Meth)acrylsäure in 4 bis 16 Portionen. In diesem Zusammenhang kann auch von sukzessiven Verfahrensstufen gesprochen werden. Die erste Zudosierung von (Meth)acrylsäure ist in diesem Sinne die erste Verfahrensstufe, die zweite Zudosierung von (Meth)acrylsäure ist dann die zweite Verfahrensstufe, usw.

In einer Ausführungsform legt man nach Zugabe jeder (Meth)acrylsäure-Portion einen Vakuumgradienten derart an, dass die Reaktionsmischung kontinuierlich siedet.
In einer Ausführungsform entfernt man das bei der Reaktion entstehende Wasser kontinuierlich aus dem Reaktionsraum.

In einer Ausführungsform dosiert man die jeweils nachfolgende (Meth)acrylsäure-Portion erst dann zu, wenn die Säurezahl des Reaktionsgemisches unter einen Wert von 100 mg KOH/g gefallen ist. Dabei ist es besonders bevorzugt, wenn die Säurezahl des Reaktionsgemisches bei der nachfolgenden Verfahrensstufe gleich oder höher ist als bei der vorherigen Verfahrensstufe. In der letzten Verfahrensstufe stellt man die Säurezahl vorzugsweise niedriger ein als die Säurezahl der vorletzten Verfahrensstufe; dies hat den Vorteil, dass dadurch die nachgeschalteten alkalischen Waschstufen verkürzt werden, zumal die Säurezahl im fertigen Endprodukt vorzugsweise auf einen Wert unterhalb von 1 mg KOH/g eingestellt wird, damit das Endprodukt lagerstabil ist bzw. eine autokatalytische saure Hydrolyse des Acrylates und ein damit einhergehender Verlust der Produkteigenschaften vermieden wird.

Man dosiert die (Meth)Acrylsäure in 4 bis 16 Portionen zu. Dabei ist es besonders bevorzugt, die (Meth)Acrylsäure in jeweils gleicher Menge zuzudosieren, insbesondere jeweils in Mengen im Bereich von 5 bis 25 Mol-% und ganz besonders bevorzugt im Bereich von 10 bis 20 Mol-%.

In einer besonders bevorzugten Ausführungsform beträgt die Gesamtmenge der zudosierten (Meth)acrylsäure-Portionen 105 bis 160 mol-% - bezogen auf die Gesamtheit der OH-Gruppen der eingesetzten Polyole.

Unter Polyolen werden im Rahmen der vorliegenden Anmeldung organische Substanzen verstanden, die zwei oder mehr OH-Gruppen pro Molekül aufweisen. Bei den einzelnen OH-Gruppen der Polyole kann es sich - jeweils unabhängig voneinander - um primäre, sekundäre oder tertiäre OH-Gruppen handeln. Als Polyole eignen sich beispielsweise aliphatische, cycloaliphatische oder aromatische Polyhydroxyverbindungen. Beispiele für geeignete Polyole sind etwa: Glycerin, Trimethylolpropan, Tripropylenglykol, Dipropylenglykol, 1.4-Cyclohexandimethanol, Tricyclodekandimethanol, Neopentylglykol, 3-Methyl-1.5-Pentandiol, 1.6-Hexandiol, 1.10-Dekandiol, Polyethylengykole mit unterschiedlichen Molekulargewicht, Polypropylengykole mit unterschiedlichen Molekulargewicht, Pentaerythrit, Dipentaerythrit, Ditrimethylolpropan, Diglycerin, Triglycerin, Polyglycerine mit unterschiedlichen Molekulargewicht sowie entsprechend ethoxylierte und/oder propoxylierte Derivate

Neben den OH-Gruppen können die Polyole gegebnenfalls auch weitere funktionelle Gruppen enthalten, insbesondere solche, die unter den Reaktuionsbedingungen inert sind, beispielsweise Polyetherpolyole oder Polyurethanpolyole.

Als Veresterungskatalysatoren werden starke organische oder anorganische Säuren eingesetzt, die einen pKa Wert von 2.5 oder weniger aufweisen. Typische Beispiele für starke organische Säuren sind etwa Methansulfonsäure und p-Toluolsulfonsäure; Beispiele für starke anorganische Säuren sind etwa Schwefelsäure und Phosphorsäure. Es können aber auch stark saure lonenaustauscherharze und Zeolithe eingesetzt werden.

Als geeignete Polymerisationsinhibitoren können beispielsweise Chinone, Alkylphenole, Alkoxyphenole und Phenothiazine eingesetzt werden, wobei 4-Methoxyphenol besonders bevorzugt ist. Weitere Beispiele für geeignete Polymerisationsinhibitoren können der WO-A-2009/106550 entnommen werden.

Die Reaktionstemperatur liegt wie bereits ausgeführt im Bereich von 70 bis 150 °C. Vorzugsweise arbeitet man im Bereich von 80 bis 120°C.

Wie bereits ausgeführt wird das bei der Reaktion entstehende Wasser unter reduziertem Druck, worunter ein Druck von 600 hPa bar oder weniger verstanden wird, aus dem Reaktionsraum entfernt. Vorzugsweise arbeitet man bei Drucken von von 400 hPa bar oder weniger.

Durch Einstellung eines geeigneten Vakuumgradienten wird bewirkt, dass das bei der Veresterung gebildete Reaktionswasser schnell und effektiv ausgeschleust wird. Das Reaktionswasser destilliert dabei als Wasser-(Meth)Acrylsäure-Gemisch ab, wobei vorzugsweise in Reaktoren gearbeitet wird, die mit Dephlegmatoren oder Destillationskolonnen ausgerüstet sind. Je nach Trennleistung und Rücklaufverhältnis wird über Dephlegmatoren oder Destillationskolonnen eine Dampfphase abgezogen, die mit dem niedriger siedenden Wasser angereichert ist, während der flüssige Rücklauf mit der höher siedenden (Meth)Acrylsäure angereichert ist. Auf diesem Wege werden die jeweils auftretenden Verdampfungsverluste an (Meth)Acrylsäure minimiert und der Rohstoffeinsatz optimiert.

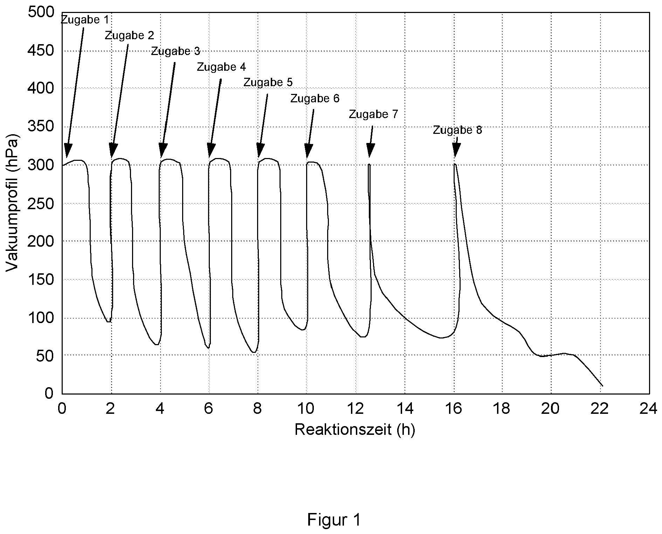

In einer bevorzugten Ausführungsform werden die Polyole, der Polymerisationsinhibitor sowie der saure Katalysator unter Durchleiten von Luft in einem Reaktor vorgelegt und dann die erste Portion an (Meth)acrylsäure zudosiert, wobei man die gesamte Zugabemenge an (Meth)acrylsäure vorzugsweise auf einen Wert im Bereich von 105 - 160 Mol% einstellt.
Nach Reduktion des Druckes und Aufheizen auf die Reaktionstemperatur wird ein Vakuumgradient (siehe Figur 1) angelegt, so dass die Reaktionsmischung über eine festgelegte Zeitdauer kontinuierlich am Sieden gehalten wird, um dem Reaktionsgemisch das Reaktionswasser möglichst vollständig zu entziehen. Vorzugsweise wird die jeweils nachfolgende (Meth)acrylsäureportion erst dann zugegeben, also die nächste Verfahrensstufe eingeleitet, wenn die Säurezahl des Reaktionsgemisches unter einen Wert von 100 mg KOH/g gefallen ist. Vor Einleiten der letzten Verfahrensstufe sollte die Säurezahl vorzugsweise unter einen Wert von 50 mg KOH/g gefallen sein sollte.

Der die erste Verfahrensstufe geschilderte Ablauf wird anschließend auch für die weiteren Verfahrensstufen wiederholt. Optional kann in einer dieser nachfolgenden Prozessstufen sowohl weiterer saurer Katalysator als auch Polymerisationsinhibitor zugefügt werden.

Die Aufarbeitung der Reaktionsmischung kann beispielsweise durch Neutralisationion, Waschen und Filtration nach allen dem Fachmann einschlägig bekannten Methoden erfolgen.

### Beispiele

### 1. Eingesetzte Substanzen

**Acrylsäure** - BASF SE (CAS-Nummer 79-10-7, Molekulargewicht: 72.06 g/mol)
**Tripropylenglykol** - LyondellBasell (CAS-Nummer 24800-44-0, Molekulargewicht:
192.26 g/mol)
**Methansulfonsäure** (70 Gew.-%) - BASF SE (CAS-Nummer 75-75-2, Molekulargewicht: 96.10 g/mol)
**Schwefelsäure** (95 Gew.-%) - Quaron France (CAS-Nummer 7664-93-9, Molekulargewicht: 98.08 g/mol)
**Phosphinsäure** (50 Gew.-%) - Minakem S.A.S (CAS-Nummer 6303-21-5, Molekulargewicht: 66.00 g/mol)
**4-Methoxyphenol** - Acros Chimica (CAS-Nummer 150-76-5, Molekulargewicht: 124.14 g/mol)
**Natriumcarbonat wasserfrei** - Quaron France (CAS-Nummer 497-19-8, Molekulargewicht: 105.99 g/mol)
**Natriumcarbonat Decahydrat** - Disachim S.A. (CAS-Nummer 6132-02-1, Molekulargewicht: 286.14 g/mol)
**Natriumsulfat wasserfrei** - Brenntag N.V. (CAS-Nummer 7757-82-6, Molekulargewicht: 142.04 g/mol)
**Dicalite 4158** (natürliches Natrium/Kalium/Aluminiumsilikat) - Dicalite Europe N.V. (CAS-Nummer 93763-70-3)

### 2. Mess- und Prüfmethoden

- Säurezahl: gemäß Norm NF EN ISO 660
- Wassergehalt: gemäß Norm ISO 4317
- Farbe APHA: gemäß Norm ISO 6271
- Viskosität: gemäß Norm ISO 2555
- Gaschromatographie:
   Gaschromatograph: 430-GC von Varian
   Säule: CP-Sil 8 CB (10 m Länge, 0.15 mm Innendurchmesser, Filmschichtdicke 0.12 µm) von Agilent Technologies
   Trägergas: Helium
   Injektionsvolumen: 5.0 µl
   Split Injektion: 1 : 100
   Detektor: Flammenionisation
   Injektortemperatur: 300 °C
   Detektortemperatur: 350 °C
   Temperaturprogramm: 120 °C für 2 Minuten, Aufheizen 120-300 °C mit 20 °C pro Minute / 300 °C für 5 Minuten
   Probenvorbereitung: keine (direkte Einspritzung)
   Peakzuordnung Gaschromatographie:

Im Folgenden werden die Retentionszeiten einzelner Substanzen zusammen mit deren Struktur und der chemischen Bezeichnung angegeben:
Retentionszeit = 3.6 Minuten 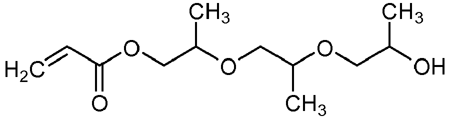 Tripropylenglykol monoacrylat
Retentionszeit = 5.2 Minuten 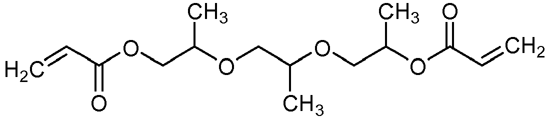 Tripropylenglykoldiacrylat
Retentionszeit = 6.0 Minuten 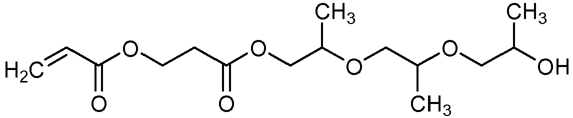 Michael-Addukt Tripropylenglykolmonoacrylat/Acrylsäure
Retentionszeit = 7.0 Minuten 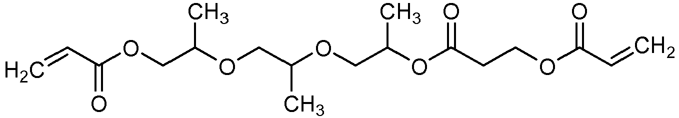 Michael-Addukt Tripropylenglykoldiacrylat/Acrylsäure
Retentionszeit = 9.3 Minuten 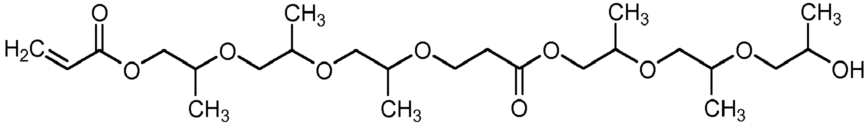 Michael-Addukt Tripropylenglykolmonoacrylat/Tripropylenglykolmonoacrylat
Retentionszeit = 10.0 Minuten 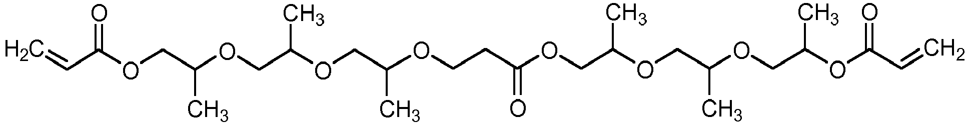 Michael Addukt Tripropylenglykoldiacrylat/Tripropylenglykolmonoacrylat

### 3. Ausführungsbeispiele

### Beispiel 1 (erfindungsgemäß)

In einem 25 m³ Reaktor wurden unter Durchleiten eines konstanten Luftstromes und bei 45 °C Reaktortemperatur 10400 kg (54093 mol) Tripropylenglykol vorgelegt und dann ein Vakuum von 300 hPa eingestellt.

In der ersten Stufe wurden nacheinander unter Rühren 15 kg (121 mol) 4-Methoxyphenol, 1197 kg (16611 mol) Acrylsäure, 188 kg (1424 mol) 50 Gew.-%ige Phosphinsäure und 188 kg (1821 mol) 95 Gew.-%ige Schwefelsäure zugegeben. Anschließend wurde die Temperatur des Reaktionsgemisches auf 90 °C erhöht. Unter diesen Temperatur/Druckbedingungen setzte die direkte Veresterung ein, wobei kontinuierlich eine Mischung aus Reaktionswasser und Acrylsäure entsprechend der Zusammensetzung der Siedekurve des betreffenden Phasengleichgewichts-zustandes abdestillierte. Nach 1 h wurde der Druck bei konstanter Temperatur des Reaktionsgemisches schrittweise gesenkt zunächst in 0.25 h auf 150 hPa und schließlich in 0.75 h auf 100 hPa. Der Druck von 100 hPa wurde so lange gehalten, bis die Säurezahl des Reaktionsgemisches unter 40 mg KOH/kg gefallen war. Im Anschluss daran wurde der Druck wieder auf 300 hPa angehoben.

In der zweiten Stufe wurden 1197 kg (16611 mol) Acrylsäure zugesetzt, der Druck 0.75 h bei 300 hPa gehalten, dann über 0.15 h auf 130 hPa gesenkt und schließlich über 1 h auf 70 hPa heruntergefahren. Der Druck von 70 hPa wurde so lange gehalten, bis die Säurezahl des Reaktionsgemisches unter 50 mg KOH/kg gefallen ist. Im Anschluss daran wurde der Druck wieder auf 300 hPa angehoben.

In der dritten Stufe wurden 1197 kg (16611 mol) Acrylsäure zugesetzt, der Druck 0.85 h bei 300 hPa gehalten, dann über 0.15 h auf 200 hPa gesenkt und schließlich über 1 h auf 60 hPa heruntergefahren. Der Druck von 60 hPa wurde so lange gehalten, bis die Säurezahl des Reaktionsgemisches unter 60 mg KOH/kg gefallen war. Im Anschluss daran wurde der Druck wieder auf 300 hPa angehoben.

In der vierten Stufe wurden 1197 kg (16611 mol) Acrylsäure zugesetzt, der Druck 0.85 h bei 300 hPa gehalten, dann über 0.15 h auf 120 hPa gesenkt und schließlich über 1 h auf 60 hPa heruntergefahren. Der Druck von 60 hPa wurde so lange gehalten, bis die Säurezahl des Reaktionsgemisches unter 60 mg KOH/kg gefallen ist. Im Anschluss daran wurde der Druck wieder auf 300 hPa angehoben.

In der fünften Stufe wurden 1197 kg (16611 mol) Acrylsäure zugesetzt, der Druck 0.85 h bei 300 hPa gehalten, dann über 0.15 h auf 120 hPa gesenkt und schließlich über 1 h auf 90 hPa heruntergefahren. Der Druck von 90 hPa wurde so lange gehalten, bis die Säurezahl des Reaktionsgemisches unter 70 mg KOH/kg gefallen ist. Im Anschluss daran wurde der Druck wieder auf 300 hPa angehoben.

In der sechsten Stufe wurden 1197 kg (16611 mol) Acrylsäure sowie 126 kg (918 mol) 70 Gew.-%ige Methansulfonsäure zugesetzt, der Druck 0.5 h bei 300 hPa gehalten, über 0.35 h auf 250 hPa gesenkt, über 0.15 h auf 140 hPa und schließlich über 1.5 h auf 80 hPa heruntergefahren. Der Druck von 80 hPa wurde so lange gehalten, bis die Säurezahl des Reaktionsgemisches unter 80 mg KOH/kg gefallen war. Im Anschluss daran wurde der Druck wieder auf 300 hPa angehoben.
In der siebten Stufe wurden 1197 kg (16611 mol) Acrylsäure zugesetzt, der Druck 0.1 h bei 300 hPa gehalten, dann über 0.4 h auf 140 hPa gesenkt und schließlich über 3 h auf 80 hPa heruntergefahren. Der Druck von 80 hPa wurde so lange gehalten, bis die Säurezahl des Reaktionsgemisches unter 100 mg KOH/kg gefallen war. Im Anschluss daran wurde der Druck wieder auf 300 hPa angehoben.

In der achten und letzten Stufe wurden 1197 kg (16611 mol) Acrylsäure zugesetzt, der Druck 0.1 h bei 300 hPa gehalten, über 0.9 h auf 130 hPa gesenkt, über 1.7 h auf 80 hPa gesenkt, über 0.7 h auf 50 hPa gesenkt, für 1.5 h gehalten und schließlich über 1.2 h auf 10 hPa heruntergefahren. Der Druck von 10 hPa wurde so lange gehalten, bis die Säurezahl des Reaktionsgemisches unter 30 mg KOH/kg gefallen war. Danach wurde auf Atmosphärendruck eingestellt.

Figur 1 zeigt den eingestellten Druck über der Reaktionszeit, mit
Zugabe 1: 15.35 mol% Acrylsäure
Zugabe 2: 15.35 mol% Acrylsäure
Zugabe 3: 15.35 mol% Acrylsäure
Zugabe 4: 15.35 mol% Acrylsäure
Zugabe 5: 15.35 mol% Acrylsäure
Zugabe 6: 15.35 mol% Acrylsäure
Zugabe 7: 15.35 mol% Acrylsäure
Zugabe 8: 15.35 mol% Acrylsäure
Reaktionstemperatur: 90 °C

Die so erhaltenen 16623 kg Rohprodukt wurden auf 55 °C abgekühlt und dann unter Rühren in 3302 kg einer 15 Gew.-%igen Lösung von wasserfreiem Natriumcarbonat in demineralisiertem Wasser eingetragen, die ebenfalls auf 55°C eingestellt war. Unter Rühren wurden 200 kg Natriumcarbonat Decahydrat langsam zugegeben und 2 h bei 55 °C weitergerührt. Danach wurde 3 h bei 55 °C ohne Rühren stehen gelassen und anschließend die wässrige Phase abgetrennt. Der Wassergehalt des verbleibenden 15312 kg Rohprodukts wurde zu 2.3% bestimmt. Danach wurde das Rohprodukt unter Rühren bei 55 °C unter Eintragen von 3652 kg einer 8.4 Gew.-%igen Lösung von wasserfreiem Natriumsulfat in demineralisiertem Wasser nachgewaschen und 0.75 h bei dieser Temperatur weitergerührt. Dann wurde 5 h bei 55 °C ohne Rühren stehen gelassen und anschließend die wässrige Phase abgetrennt. Das verbleibende Rohprodukt wurde unter Rühren und Durchleiten von Luft bei 55 °C unter Vakuum getrocknet, wobei im ersten Schritt der Druck in 3.5 h von 1013 hPa auf 170 hPa gesenkt wurde, gefolgt von 0.7 h auf 90 hPa und schließlich von 0.9 h auf 10 hPa. Zur abschließenden Reinigung und Filtration wurde das Rohprodukt unter Rühren auf 40 °C abgekühlt, mit dem Filtrationshilfsmittel Dicalite 4158 versetzt und dann über eine Filterpresse geschickt. Es resultierten 15000 kg Endprodukt mit folgenden analytischen Daten:
Säurezahl → 0.15 mg KOH/g
Wassergehalt → 0.15 Gew.-%
Farbe APHA → 36
Viskosität (25.0 °C) → 14 mPa·s

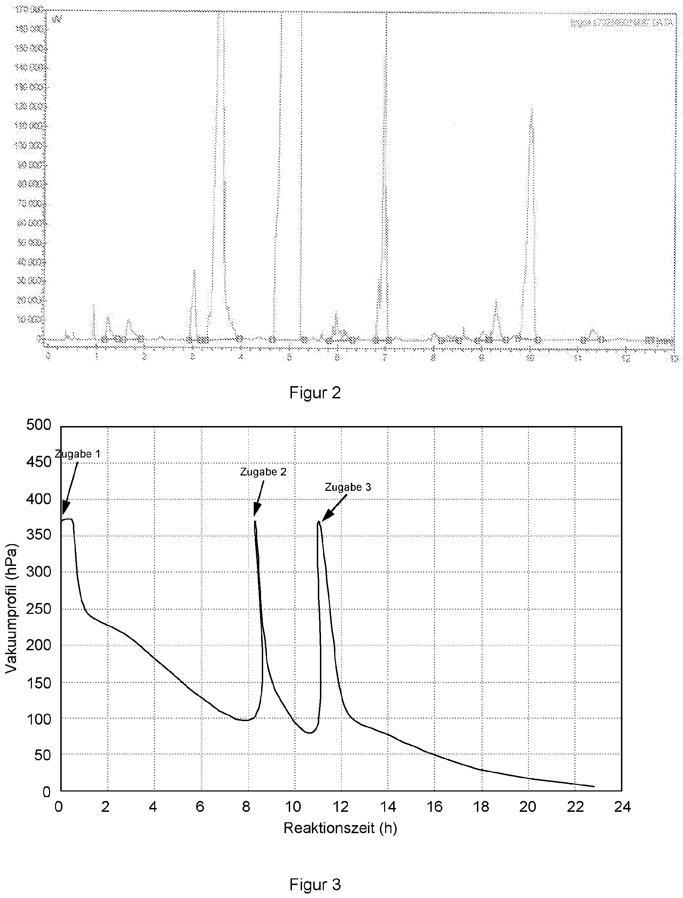

Die GC-Analyse des Endproduktes (Figur 2) ergab folgende Zusammensetzung (GC-Flächenprozent):
Tripropylenglykolmonoacrylat: 9.582%
Tripropylenglykoldiacrylat: 80.873%

### Michael-Addukte:

- Tripropylenglykolmonoacrylat/Acrylsäure: 0.379%
- Tripropylenglykoldiacrylat/Acrylsäure: 2.957%
- Tripropylenglykolmonoacrylat/Tripropylenglykolmonoacrylat 0.508%
- Tripropylenglykoldiacrylat/Tripropylenglykolmonoacrylat: 4.160%
Rest: 1.541 %

### Veraleichsbeispiel

In einem 25 m³ Reaktor wurden unter Durchleiten eines konstanten Luftstromes und bei 45 °C Reaktortemperatur 10400 kg (54093 mol) Tripropylenglykol vorgelegt und der Druck auf 400 hPa gesenkt. Dann wurden nacheinander unter Rühren 15 kg (121 mol) 4-Methoxyphenol, 7768 kg (107799 mol) Acrylsäure, 188 kg (1424 mol) 50 Gew.-%ige Phosphinsäure und 188 kg (1821 mol) 95 Gew.-%ige Schwefelsäure zugegeben. Anschließend wurde die Temperatur des Reaktionsgemisches auf 85 °C erhöht und der Druck auf 370 hPa gesenkt.

Unter diesen Temperatur- und Druckbedingungen setzte die direkte Veresterung ein, wobei kontinuierlich eine Mischung aus Reaktionswasser und Acrylsäure entsprechend der Zusammensetzung der Siedekurve des betreffenden Phasengleichgewichtszustandes abdestillierte. Nach 0.5 h wurde der Druck bei konstanter Temperatur des Reaktionsgemisches schrittweise reduziert zunächst in 0.5 h auf 250 hPa, dann in 0.75 h auf 215 hPa und schließlich in 5.5 h auf 100 hPa. Der Druck von 100 hPa wurde so lange gehalten, bis die Säurezahl des Reaktionsgemisches unter 140 mg KOH/kg gefallen war. Im Anschluss daran wurde der Druck wieder auf 370 hPa angehoben und weitere 904 kg (12545 mol) Acrylsäure sowie 126 kg (918 mol) 70 Gew.-%ige Methansulfonsäure zugegeben. In der Folge wurde der Druck wieder schrittweise gesenkt in 0.75 h auf 155 hPa und dann in 2 h auf 90 hPa, der so lange gehalten wurde, bis die Säurezahl des Reaktionsgemisches unter 120 mg KOH/g gesunken war. Dann wurde der Druck wieder auf 370 hPa angehoben und weitere 904 kg (12545 mol) Acrylsäure zugegeben. Zum Abschluss wurde der Druck erneut schrittweise abgesenkt auf 120 hPa in 1.1 h, auf 80 hPa in 1.7 h, dann auf 30 hPa in 4 h und schließlich auf 5 hPa in 2.45 h. Der Druck von 5 hPa wurde so lange gehalten, bis das Reaktionsgemisch eine Säurezahl von 30 mg KOH/g erreicht hatte. Danach wurde auf Atmosphärendruck eingestellt.

Figur 3 zeigt den eingestellten Druck über der Reaktionszeit, mit
Zugabe 1: 99.64 Mol% Acrylsäure
Zugabe 2: 11.60 Mol% Acrylsäure
Zugabe 3: 11.60 Mol% Acrylsäure
Reaktionstemperatur: 85 °C

Die erhaltenen 16623 kg Rohprodukt wurden auf 55 °C abgekühlt und dann zur Neutralisation unter Rühren in 3302 kg einer 15 Gew.-%igen Lösung von wasserfreiem Natriumcarbonat in demineralisiertem Wasser eingetragen, die ebenfalls auf 55 °C eingestellt waren. Unter Rühren wurden 200 kg Natriumcarbonat Decahydrat langsam zugegeben und 2 h bei 55 °C weitergerührt. Danach wurde 3 h bei 55 °C ohne Rühren stehen gelassen und anschließend die wässrige Phase abgetrennt. Der Wassergehalt der verbleibenden 15312 kg Rohprodukt wurde bestimmt, danach unter Rühren bei 55 °C durch Eintragen von 3652 kg einer 8.4 Gew.-%igen Lösung von wasserfreiem Natriumsulfat in demineralisiertem Wasser nachgewaschen und schließlich 0.75 h bei dieser Temperatur weitergerührt. Dann wurde 5 h bei 55 °C ohne Rühren stehen gelassen und anschließend die wässrige Phase abgetrennt. Das verbleibende Rohprodukt wurde unter Rühren und Durchleiten von Luft bei 55 °C unter Vakuum getrocknet, wobei im ersten Schritt der Druck in 3.5 h von 1013 hPa auf 170 hPa gesenkt wurde, gefolgt von 0.7 h auf 90 hPa und schließlich von 0.9 h auf 10 hPa. Zur Filtration wurde das Rohprodukt unter Rühren auf 40 °C abgekühlt, mit dem Filtrationshilfsmittel Dicalite 4158 versetzt und dann auf eine Filterpresse gegeben. Es resultierten 15000 kg Endprodukt mit folgenden analytischen Daten:
Säurezahl → 0.10 mg KOH/g
Wassergehalt → 0.20 Gew.-%
Farbe APHA → 8
Viskosität (25.0 °C) → 13 mPa·s

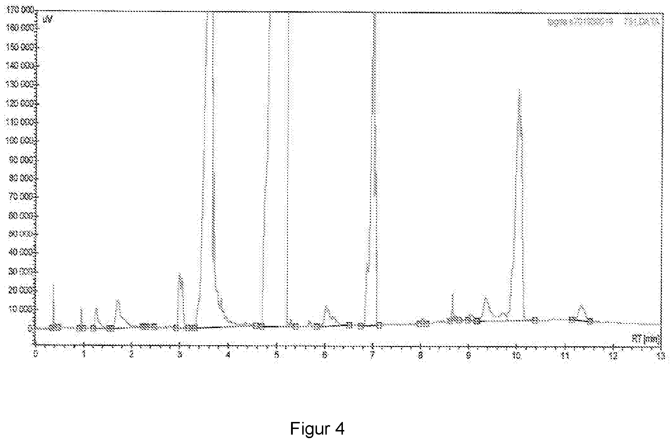

Die GC-Analyse des Endproduktes (Figur 4) ergab folgende Zusammensetzung (GC-Flächenprozent):
Tripropylenglykolmonoacrylat: 12.021%
Tripropylenglykoldiacrylat: 76.092%

### Michael-Addukte:

- Tripropylenglykolmonoacrylat/Acrylsäure: 0.461%
- Tripropylenglykoldiacrylat/Acrylsäure: 4.348%
- Tripropylenglykolmonoacrylat/Tripropylenglykolmonoacrylat: 0.637%
- Tripropylenglykoldiacrylat/Tripropylenglykolmonocrylat: 4.435%
Rest: 2.006%

## Patentansprüche

1. Verfahren zur Herstellung von (Meth)acrylsäureestern von Polyolen, wobei der Gehalt dieser Ester an Species, bei denen sämtliche OH-Gruppen der Polyole verestert sind, 80 Mol% oder mehr beträgt, durch Umsetzung von Polyolen mit Acrylsäure und/oder Methacrylsäure in Gegenwart von sauren Veresterungskatalysatoren und in Gegenwart von Polymerisationsinhibitoren, wobei man mit bei Reaktionstemperatur flüssigen Reaktionsgemischen arbeitet, die frei von nicht reagierenden Lösungs- und/oder azeotropen Schleppmitteln sind, wobei das entstehende Kondensationswasser aus der Gasphase des Reaktionsraumes abgezogen wird, **dadurch gekennzeichnet, dass** man (Meth)Acrylsäure in 4 bis 16 Portionen zudosiert, wobei folgende Maßgaben gelten:
• die Menge der einzelnen (Meth)acrylsäure-Portionen wird jeweils im Bereich von 5 bis 40 Mol-% - bezogen auf die Gesamtheit der OH-Gruppen der eingesetzten Polyole - eingestellt,
• die Anzahl der (Meth)acrylsäure-Portionen multipliziert mit der Menge der eingesetzten (Meth)acrylsäure-Portionen (in Mol%) ergibt einen Wert von mindestens 100 (Mol%),
• die Reaktionstemperatur wird auf einen Wert im Bereich von 70 bis 150 °C eingestellt und
• das bei der Reaktion entstehende Wasser wird unter reduziertem Druck aus dem Reaktionsraum entfernt, wobei der reduzierte Druck bei 600 hPa oder weniger liegt.

2. Verfahren nach Anspruch 1, wobei man die (Meth)Acrylsäure in jeweils gleicher Menge zudosiert.

3. Verfahren nach Anspruch 1 oder 2, wobei man nach Zugabe jeder (Meth)acrylsäure-Portion einen Vakuumgradienten derart anlegt, dass die Reaktionsmischung kontinuierlich siedet.

4. Verfahren nach einem der Ansprüche 1 bis 3, wobei man das bei der Reaktion entstehende Wasser kontinuierlich aus dem Reaktionsraum entfernt.

5. Verfahren nach einem der Ansprüche 1 bis 4, wobei die jeweils nachfolgende (Meth)acrylsäure-Portion erst dann zugegeben wird, wenn die Säurezahl des Reaktionsgemisches unter einen Wert von 100 mg KOH/g gefallen ist.

6. Verfahren nach Anspruch 5, wobei die Säurezahl des Reaktionsgemisches bei der nachfolgenden Verfahrensstufe gleich oder höher ist als bei der vorherigen Verfahrensstufe, mit Ausnahme der letzten Verfahrensstufe, deren Säurezahl niedriger als die Säurezahl der vorletzten Verfahrensstufe liegt.

7. Verfahren nach einem der Ansprüche 1 bis 6, wobei die Gesamtmenge der zudosierten (Meth)acrylsäure-Portionen 105 bis 160 mol-% - bezogen auf die Gesamtheit der OH-Gruppen der eingesetzten Polyole - beträgt.

## Claims

1. A process for preparing (meth)acrylic esters of polyols, the amount in these esters of species in which all of the OH groups of the polyols are esterified being 80 mol% or more, by reaction of polyols with acrylic acid and/or methacrylic acid in the presence of acidic esterification catalysts and in the presence of polymerization inhibitors, operating with reaction mixtures which are liquid at reaction temperature and are free from nonreacting solvents and/or azeotropic entrainers, the resultant water of condensation being stripped from the gas phase of the reaction space, **characterized in that** (meth)acrylic acid is metered in in 4 to 16 portions, with the following provisos:
• the amount of the individual (meth)acrylic acid portions is set in each case in the range from 5 to 40 mol%, based on the entirety of the OH groups of the polyols used,
• the number of (meth)acrylic acid portions, multiplied by the amount of (meth)acrylic acid portions used (in mol%), produces a figure of at least 100 (mol%),
• the reaction temperature is set to a level in the range from 70 to 150°C, and
• the water formed in the reaction is removed from the reaction space under reduced pressure, the reduced pressure being 600 hPa or less.

2. The process according to claim 1, wherein the (meth)acrylic acid is metered in in an equal amount in each case.

3. The process according to claim 1 or 2, addition of each (meth)acrylic acid portion being followed by application of a vacuum gradient such that the reaction mixture boils continuously.

4. The process according to claim 1 to 3, the water formed in the reaction being removed from the reaction space continuously.

5. The process according to any of claims 1 to 4, each subsequent (meth)acrylic acid portion being added only when the acid number of the reaction mixture has dropped below a level of 100 mg KOH/g.

6. The process according to claim 5, the acid number of the reaction mixture in the subsequent process stage being equal to or higher than in the preceding process stage, with the exception of the last process stage, whose acid number is lower than the acid number of the penultimate process stage.

7. The process according to any of claims 1 to 6, the total amount of the (meth)acrylic acid portions metered in being 105 to 160 mol%, based on the entirety of the OH groups of the polyols used.

## Revendications

1. Procédé de fabrication d'esters de l'acide (méth)acrylique de polyols, selon lequel la teneur de ces esters en espèces dans lesquelles tous les groupes OH des polyols sont estérifiés est de 80 % en moles ou plus, par mise en réaction de polyols avec de l'acide acrylique et/ou de l'acide méthacrylique en présence de catalyseurs d'estérification acides et en présence d'inhibiteurs de polymérisation, des mélanges réactionnels liquides à la température de réaction étant utilisés, qui sont exempts de solvants et/ou d'agents d'entraînement azéotropes non réactifs, l'eau de condensation formée étant extraite de la phase gazeuse de la chambre de réaction, **caractérisé en ce que** l'acide (méth)acrylique est dosé en 4 à 16 parties, les mesures suivantes étant appliquées :
- la quantité des parties d'acide (méth)acrylique individuelles est à chaque fois ajustée dans la plage allant de 5 à 40 % en moles, par rapport à la totalité des groupes OH des polyols utilisés,
- le nombre des parties d'acide (méth)acrylique multiplié par la quantité des parties d'acide (méth)acrylique utilisées (en % en moles) donne une valeur d'au moins 100 (% en moles),
- la température de réaction est ajustée à une valeur dans la plage allant de 70 à 150 °C, et
- l'eau formée lors de la réaction est éliminée sous pression réduite de la chambre de réaction, la pression réduite étant de 600 hPa ou moins.

2. Procédé selon la revendication 1, selon lequel l'acide (méth)acrylique est à chaque fois dosé en la même quantité.

3. Procédé selon la revendication 1 ou 2, selon lequel un gradient de vide est appliqué après l'ajout de chaque partie d'acide (méth)acrylique, de telle sorte que le mélange réactionnel soit en continu en ébullition.

4. Procédé selon l'une quelconque des revendications 1 à 3, selon lequel l'eau formée lors de la réaction est éliminée de la chambre de réaction en continu.

5. Procédé selon l'une quelconque des revendications 1 à 4, selon lequel la partie d'acide (méth)acrylique suivante n'est à chaque fois ajoutée que lorsque l'indice d'acidité du mélange réactionnel a chuté en dessous d'une valeur de 100 mg de KOH/g.

6. Procédé selon la revendication 5, selon lequel l'indice d'acidité du mélange réactionnel lors de l'étape de procédé ultérieure est supérieur ou égal à celui lors de l'étape de procédé antérieure, à l'exception de la dernière étape de procédé, dont l'indice d'acidité est inférieur à l'indice d'acidité de l'avant-dernière étape de procédé.

7. Procédé selon l'une quelconque des revendications 1 à 6, selon lequel la quantité totale des parties d'acide (méth)acrylique dosées est de 105 à 160 % en moles, par rapport à la totalité des groupes OH des polyols utilisés.
